# EUROPEAN PATENT APPLICATION

(11) **EP 0 592 805 A2**
(43) Date of publication of application: **20.04.1994**
(21) Application number: 93114278.0
(22) Date of filing: 06.09.1993
(51) Int. Cl.: G01N 27/30

(54) **Carbon sensor electrode and process for producing the same**

(30) Priority: 09.09.1992 JP 266706/92; 09.09.1992 JP 266707/92
(71) Applicant: AGENCY OF INDUSTRIAL SCIENCE & TECHNOLOGY MINISTRY OF INTERNATIONAL TRADE & INDUSTRY, Tokyo (JP)
(72) Inventor: Kaneko, Hiroko, Tsukuba-shi, Ibaraki (JP); Negishi, Akira, Matsudo-shi, Chiba (JP); Nozaki, Ken, Tsukuba-shi, Ibaraki (JP)
(74) Representative: Geyer, Ulrich F., Dr. Dipl.-Phys.

(57) **Abstract**

A carbon electrode is dipped in a solution containing a reactant (12), and the carbon electrode impregnated with the solution is covered with an insulating outer layer (11); or a solution containing a reactant is poured into an insulating tube, and a carbon electrode is inserted into the tube; whereby the reactant is made coexistent in the periphery of the carbon electrode. Alternatively, a reactant such as an active enzyme and graphite particles or activated carbon particles (13) are mixed with a liquid and dispersed therein to prepare a thoroughly wetted paste (14). The paste is introduced by means of an extruding mechanism (16, 17) such as a syringe into an insulating outer layer member in which a bundle of many thin carbon rods as a lead is inserted. Thus, a carbon sensor electrode is produced.

## Description

The present invention relates to a carbon sensor electrode for use in electrochemical detectors, sensors for environment analysis, sensors for pathological examinations, and probe electrodes for detection of in vivo conditions. It also relates to a process for producing the carbon sensor electrode.

Among major fields of electrochemical measurement is simple rapid analysis relying for detection on sensors that have shown prompt progress in recent years. Since this method is highly selective and permits high sensitivity measurement, it has found extensive use in the analysis and evaluation of clinical samples or environmental samples containing minute quantities of ingredients to be determined and having numerous compounds coexistent therein.

In recent years, it has become crucial to obtain information on such particular substances in vivo and in situ locally in a biological system by use of a sensor electrode which can detect these substances with high sensitivity and rapidity. Acquisition of that information requires the use of an easily producible disposable electrode, or the use of a new electrode which can measure a particular substance selectively in vivo.

Such measuring electrodes which have been studied and put to practical use include pH meters which measure hydrogen ions; ion sensors which detect inorganic ions such as sodium ions; sensors comprising carbon fibers or glassy carbon chemically modified with complexes or organic matters on the surfaces thereof; various enzyme sensors such as glucose sensors prepared by mixing enzymes such as glucose oxidase with the paste of a carbon paste electrode, and coating the mixture with an ion permeable membrane; and biosensors holding metabolites from immune mechanisms.

Of the above sensors, most sensors including chemically modified sensors, enzyme sensors and biosensors, except for pH meters and inorganic ion sensors, are defective in that they cannot withstand long-term use and their lives are short. Thus, a desire has been strong for the development of long life sensor electrodes preferred for use in sensors excluding pH meters and inorganic ion sensors.

The requirements for sensor electrodes include, for instance,
(i) a large potential window and a low blank current,
(ii) a highly reproducible, repeatable reaction with particular substances,
(iii) an active electrode reaction,
(iv) little differences in individual electrode characteristics,
(v) little content of impurities, not interfering an electrode reaction, and
(vi) ease of handling and pretreatment.

We, the present inventors, previously found that a probe electrode using the lead of a mechanical pencil which is a thin rod of carbon (Japanese Patent Application Laying-open No. 250854/1989) could be used as a material for a sensor electrode fulfilling most of the above-mentioned requirements, and that some carbon fibers could be used in measuring electrodes. As is well known, that mechanical pencil lead is a composite carbonaceous material comprising naturally occurring graphite and an organic binder.

We attempted to solve the aforementioned problem of life with the conventional sensors by using the composite carbonaceous material. Prior to the filing of the present application, we proposed a sensor preferred as a chemically modified sensor, an enzyme sensor or a biosensor (Japanese Patent Application Laying-open No. 18928/1993). Since micropores are formed during the production of the composite carbonaceous material, this previously proposed sensor takes advantage of the micropores to achieve increased conductivity. It also has controlled pore sizes, thereby making a sensor substrate usable for long periods. In this sensor, moreover, the surface and internal communicating micropores of a porous carbon thin wire which is the sensor substrate are impregnated, adsorbed or chemically modified with a reactant such as an enzyme, a metal complex, an organic substance or a metabolite, whereby the desired reactant is held inside or on the surface.

This electrode is a carbon sensor electrode capable of detecting particular substances, and has the following characteristics:
1. It provides a cell-scale carbon microsensor electrode which can apply an electric current, an electric voltage or a mechanical stimulus to a living system.
2. It does not poison the measuring system (if it remains in a living body, it is safe), and it can be used for testing of foods.
3. It has a mechanical strength enough to be stuck into the body or foods, permitting the electrochemical detection of a very fine (a minute amount of) portion.
4. It has minimally fluctuating characteristics, offers reproducible data, and enables reliable measurement.
5. It does not require special pretreatments, and polishing is sufficient for its stable measurement of an electrode reaction.
6. It is low-priced and disposable.

However, this electrode is disadvantageous in that the poor wettability of its carbon may pose difficulty in impregnating a hydrophilic reactant into the resulting micropores.

Furthermore, the living system where the carbon electrode is used for measurement contains large amounts of adsorbable substances such as proteins and lipids. Once adsorbed to the surface of the carbon, these organic matters are scarcely removed without oxidation. Therefore, care should be taken to discard the carbon electrode after each use, or to break off the used electrode portion. In clinical examinations, the reuse of the same sensor that has been used on other samples could cause errors for the above reason. Thus a sensor and a sensor electrode for disposable use are desired. The requirements for the sensor and sensor electrode of a construction for disposable use are as follows:
(1) Carbon friendly to the body constitutes an electrode.
(2) The carbon material has a high activity as an electrode.
(3) A reactant such as an enzyme can be easily held active in the sensor.
(4) A used electrode portion can be snapped or cut off.
(5) The price is low enough for disposable use.

A disposable sensor electrode should fulfill at least the above six requirements. For this purpose, a sensor electrode is desirable in which the desired reactant such as an enzyme, metal complex, organic substance or metabolite can be always made to exist on the surface of the electrode and which can be produced economically.

In addition to the carbon sensor electrode using the composite carbon material (lead of a mechanical pencil), a preferred sensor electrode is conceivable which is a commercially available carbon paste electrode prepared by mixing fine carbon particles with a liquid. Such a commercially available carbon paste electrode has a mixture of carbon particles for electrode and a liquid held at the tip of the electrode, or may have a reactant additionally mixed. Some of such carbon paste electrodes may be used as disposable sensors. The carbon paste electrode, however, must be handmade for each use, thus often causing delicate differences in the mixing state or in the electrode surface every time it is prepared.

The object of the present invention, therefore, is to provide the construction of a carbon sensor electrode fulfilling the above-described requirements, and a process for producing the carbon sensor electrode.

A carbon sensor electrode using a composite carbon material in accordance with the present invention comprises an insulating outer layer, a solution containing a reactant filled inside the insulating outer layer, and a carbon electrode inserted in the solution containing the reactant.

A process for producing a carbon sensor electrode using a composite carbon material in accordance with the present invention comprises dipping a carbon electrode in a solution containing a reactant, and covering the carbon electrode with an insulating outer layer with the solution being impregnated into the carbon electrode; or comprises pouring a solution containing a reactant into an insulating tube like a plastic tube, a glass tube or a ceramic tube, and inserting a carbon electrode into the tube.

The carbon electrode is preferably a bundle of many carbon fibers or thin carbon rods put together.

The insulating outer layer is preferably an insulating tube or an insulating sheet.

Examples of the reactant are organic substances including active enzymes, nitrogen metabolites such as uric acid, amino acid metabolites, neurotransmitters such as dopamine, vitamins, organic acids such as acetic acid or citric acid, amines, alcohols, and medicines such as anesthetics; complex ions; inorganic acids such as hydrochloric acid or sulfuric acid; alkalis such as ammonia or hydroxides; salts of the inorganic acids and alkalis; soluble gases such as oxygen, chlorine or nitrogen oxides; mixtures of any of these; and substances containing any of them.

To prevent the reactant from draining into the bulk solution, the reactant may be held in place with carriers such as vaseline, paraffin, oil, glycerin, agar or gelatin.

Furthermore, the tip opening of the insulating outer layer may be sealed with an ion permeable membrane, an ion exchange membrane or a dialysis membrane in order to prevent the reactant from draining into the bulk solution.

Moreover, a reaction solution feeding device for feeding a solution containing the reactant may be connected with a base portion of the insulating outer layer so that a fresh reactant can be constantly fed to the tip of the electrode. A solution injector or a pump capable of fine adjustment for a slow speed may be used as the reaction solution feeder.

A carbon sensor electrode using a carbon paste in accordance with the present invention comprises an insulating outer layer, and a carbon paste filled inside the insulating outer layer, the carbon paste comprising at least a fine carbon material and a carrier.

A process for producing the carbon sensor electrode using a carbon paste in accordance with the present invention comprises homogeneously mixing at least a fine carbon material and a carrier with a dispersing medium to prepare a carbon paste, and placing the carbon paste inside a tubular outer layer.

The fine carbon material is preferably selected from activated carbon particles, graphite particles, amorphous carbon particles, short carbon fibers, and mixtures of these.

The carrier is preferably at least one of vaseline, glycerin, oil, paraffin, agar and gelatin.

The carbon paste preferably contains a reactant. Examples of the reactant are organic substances including active enzymes, nitrogen metabolites such as uric acid, amino acid metabolites, neurotransmitters such as dopamine, vitamins, organic acids such as acetic acid or citric acid, amines, alcohols, and medicines such as anesthetics; complex ions; inorganic acids such as hydrochloric acid or sulfuric acid; alkalis such as ammonia or hydroxides; salts of the inorganic acids and alkalis; soluble gases such as oxygen, chlorine or nitrogen oxides; mixtures of any of these; and substances containing any of them.

The dispersing medium for mixing the reactant and the fine carbon material is preferably either an aqueous solution, an organic solvent, or a mixture of these.

The insulating outer layer is preferably an insulating tube or an insulating sheet.

Furthermore, the tip opening of the insulating outer layer may be sealed with an ion permeable membrane, an ion exchange membrane or a dialysis membrane.

Moreover, a carbon paste feeder capable of continuously feeding the carbon paste may be connected with a base end portion of the insulating outer layer so that a fresh sensor end can be constantly obtained.

The above and other objects, effects, features and advantages of the present invention will become more apparent from the following description of embodiments thereof taken in conjunction with the accompanying drawings.
Fig. 1 is a perspective view showing the outlined structure of a sensor electrode using a composite carbon material in accordance with the present invention;
Fig. 2 is a conceptual view illustrating the reaction in the sensor electrode;
Fig. 3 is linear sweep voltammograms (LSV) of the results of the reaction in the sensor electrode;
Fig. 4 is a simplified block diagram showing the sensor electrode using a carbon paste in accordance with the present invention;
Fig. 5 is an enlarged constructional view showing the portion II in Fig. 4;
Fig. 6 is voltammograms of uric acid in the sensor electrode using a carbon paste in accordance with the present invention without a built-in reactant; and
Fig. 7 is a graph showing cyclic voltammograms of iron-EDTA by the sensor electrode using a carbon paste in accordance with the present invention, as well as the results of detection of hydrogen peroxide by the sensor electrode.

### Embodiment 1

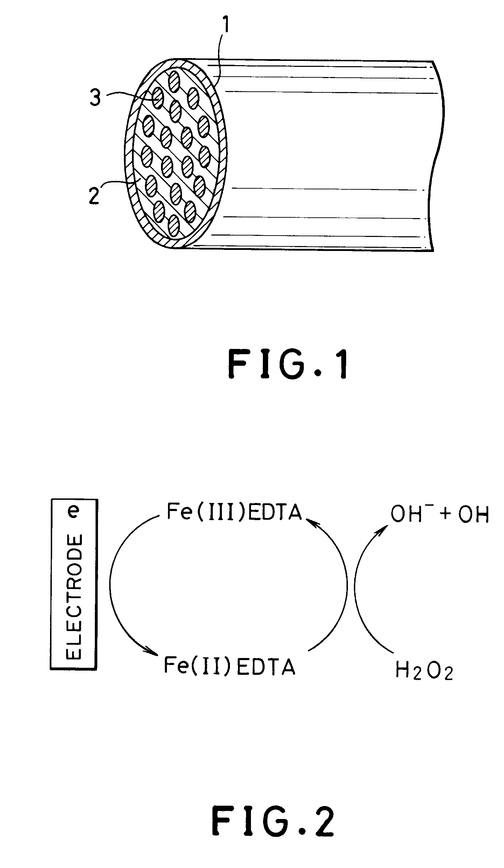

The carbon sensor electrode using a composite carbon material of the present invention, as Fig. 1 shows its outlined structure, comprises an insulating tube 1 as an outer layer, a reactant 2 held inside, and carbon of varying shapes and types as an electrode material 3 penetrating through the reactant 2. The reactant 2 is an active enzyme, a metabolite, a complex ion, an acid, an alkali, a dissolved gas or an organic substance. The top opening of the tube 1 is sealed, or the reactant 2 held inside the tube 1 is coexistent with a retaining material such as vaseline, glycerin, oil, paraffin, agar or gelatin so that the reactant 2 does not drain into the bulk solution immediately during the reaction. A tip portion of the tube 1 may be covered with an ion exchange membrane with a low electric resistance or a conductive polymeric membrane to prevent the solution from draining. Furthermore, a reaction solution feeder such as a syringe (not shown) may be connected with a base end portion of the tube 1 so that a fresh reaction solution will be constantly fed to the tip of the electrode even if an outflow of the solution occurs.

The carbon sensor electrode can be produced in a simple manner.

In case of the retaining substance being agar, a first process for production comprises dipping the electrode material 3, say, made of a bundle of carbon fibers in a hot solution of the reactant 2 dissolved suitably together with agar to wet the peripheral portion of the electrode material 3 sufficiently with the reactant 2; and then passing the electrode material 3 through the tube 1 as such or with the use of a needle-like guide. This will result in a sensor in which at room temperature the reactant does not flow out because of the viscous retaining substance.

A second process for production comprises sucking a hot solution of the reactant 2, dissolved together with the retaining substance, into the insulating tube 1 attached to the tip of a pipette; inserting a carbon material, which will serve as the electrode material 3, into the solution; and bringing it up and down therein to wet the surface of the electrode sufficiently with the reactant 2. When the reactant varying in characteristics upon heating is to be handled, the solution of the reactant is introduced at room temperature by any of the above-described methods, whereafter the tip portion of the tube 1 may be covered with an ion exchange membrane with a low electric resistance or a conductive polymeric membrane to prevent the outflow of the solution.

The tip of the carbon sensor electrode that has been used once is snapped or cut off, and a fresh electrode surface is used without fail. This will give results with high reproducibility.

In the substantial absence of the retaining substance (carrier), the top opening of the tube 1 including the electrode is sealed, or a solution injector or a pump capable of fine adjustment for a low speed is connected with the tube 1 to produce a slightly negative pressure therein and hold the reactant-containing solution around the electrode. This will make the product usable as a sensor.

The carbon sensor electrode of the present invention with the above-described construction is a long-life one comprising an insulating outer layer, a reactant such as an enzyme accommodated inside, and carbon fibers as an electrode. Since the tip of the electrode can be easily removed, a fresh sensor electrode surface can be easily provided, thus making the carbon sensor electrode of the invention highly practical.

The present embodiment will be described in greater detail below.

Iron-EDTA complex ions serving as an imitation peroxidase were placed in an insulating silicone tube with an inside diameter of 1 mm. A bundle of mechanical pencil leads with a diameter of 0.2 mm was inserted as an electrode material into the tube to obtain a sensor electrode. This sensor electrode was used to detect hydrogen peroxide present in a bulk solution. This reaction proceeds catalytically according to the following formulae, and thus has increased sensitivity and selectivity. A conceptual view of this reaction is shown in Fig. 2.

Fe(III)EDTA + e → Fe(II)EDTA (I)

Fe(II)EDTA + H₂O₂ → Fe(III)EDTA (II)

The reaction of formula (I) proceeds stably in the pH range of from 3.5 to 5 where an acetate-buffer solution is used. In this pH range, the reaction of formula (II) is much faster than the reaction of formula (I). In the presence of hydrogen peroxide, it is an enzyme-like reaction in which Fe(III)EDTA is reproduced instantaneously.

The reaction of formula (I) can be detected by voltammetry as an Iron-EDTA wave at about -0.3V vs. SCE (slightly different according to pH). If hydrogen peroxide is present, its wave height increases with increasing amount of hydrogen peroxide, thus making the detection of hydrogen peroxide possible. At this time, if there is dissolved oxygen in the solution, oxygen also performs a similar reaction to the reaction of formula (II). Hence, oxygen contained in the solution should be removed before insertion of the present sensor electrode by blowing an inert gas or the like for about 10 minutes beforehand. The tip of the instant sensor electrode was cut off with scissors, and the detection of hydrogen peroxide was attempted repeatedly. The relative standard deviation for the height of the Iron-EDTA reduction wave was 4.6% upon ten repeats of measurements.

### Embodiment 2

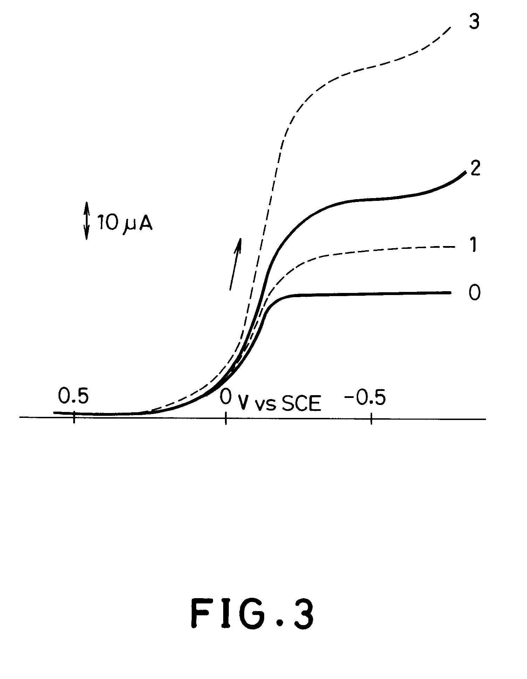

In many natural enzyme reactions, hydrogen peroxide is generated after completion of the reaction. Thus, the progress of the enzyme reaction can be estimated by measuring the content of hydrogen peroxide in the reaction solution. In the present invention, the final objective is a sensor electrode of the present construction incorporating peroxidase contained in natural wasabi (a Japanese horseradish). However, the use of Iron-EDTA utilized in Embodiment 1 facilitates the detection of hydrogen peroxide as described above. When it was attempted to detect 1 to 5 mM of hydrogen peroxide present in the solution at pH 4.5, the results shown in Fig. 3 were obtained, confirming the high characteristics of the sensor electrode of the present invention. Curve 0 is a linear sweep voltammogram (LSV) only by Iron-EDTA in the sensor. Curve 1 is an LSV obtained when Iron-EDTA was present together with 0.75% H₂O₂. Curve 2 is an LSV obtained when Iron-EDTA was present together with 1.0% H₂O₂. Curve 3 is an LSV obtained when Iron-EDTA was present together with 1.5% H₂O₂.

### Embodiment 3

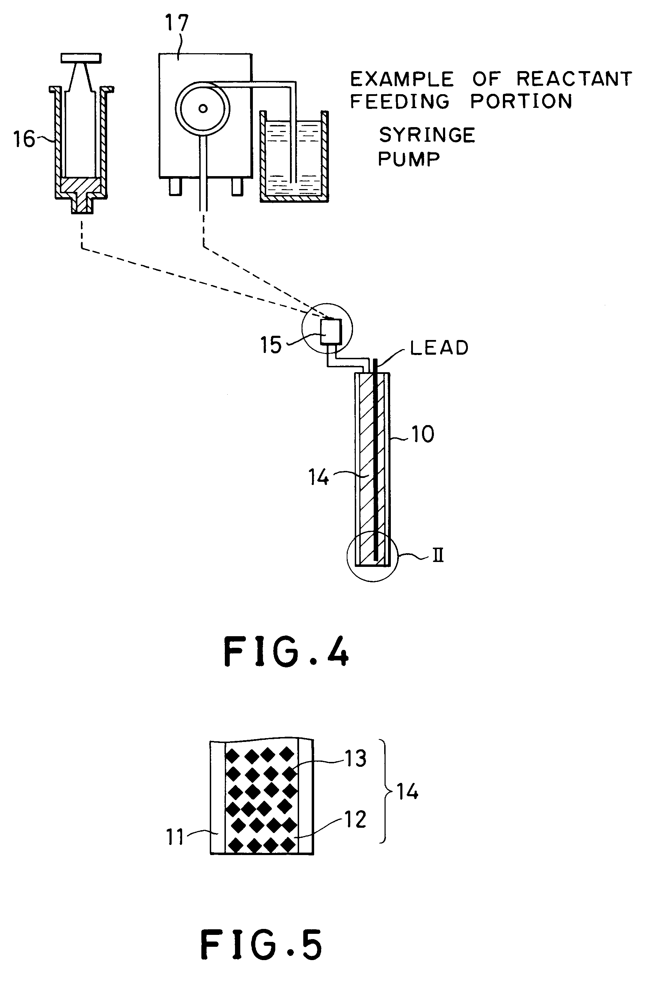

The carbon sensor electrode using a carbon paste of the present invention, as Fig. 1 shows its outlined structure, has an insulating tube as an outer layer 11. As shown in Fig. 5, this tube is charged with a carbon paste 14 formed by mixing a reactant 12, a carbon material 13 and a retaining substance (carrier). This electrode is either used with the sensor portion facing upward, or used as a suspension type.

Examples of the reactant 12 are organic substances including active enzymes, nitrogen metabolites such as uric acid, amino acid metabolites, neurotransmitters such as dopamine, vitamins, organic acids such as acetic acid or citric acid, amines, alcohols, and medicines such as anesthetics; complex ions; inorganic acids such as hydrochloric acid or sulfuric acid; alkalis such as ammonia or hydroxides; salts of the inorganic acids and alkalis; soluble gases such as oxygen, chlorine or nitrogen oxides; mixtures of any of these; and substances containing any of them.

To prevent any such reactant 12 from immediately draining into the bulk solution during the reaction, the reactant 12 may be present together with a carrier such as vaseline, glycerin, oil, paraffin, agar or gelatin.

Furthermore, a tip portion of the tube may be covered with an ion exchange membrane with a low electrical resistance, a conductive polymeric film, or a dialysis membrane in order to prevent the outflow of the solution.

A method of preparing this electrode comprises mixing the reactant 12 thoroughly with the carbon material 13 (comprising fine carbon particles or short carbon fibers) as a dispersion in water or an organic solvent along with vaseline, glycerin, oil, paraffin, agar or gelatin for holding the reactant 12 in place so as to prevent its immediate outflow into the solution. This mixing results in the formation of a carbon paste 14. Then, this carbon paste 14 is pushed into an insulating tube (outer layer 11) using a piston or the like, or is rolled in a sheet of an insulating film (outer layer 11).

With the electrode of the present invention, a sensor free from outflow of the reactant can be constructed because of the viscous retaining substance.

With the electrode of the present invention, moreover, the sensor portion (tip of the electrode) once used is snapped or cut off, and a fresh electrode surface must be used without fail; alternatively, the paste in the tip portion that has been used is pushed off to supply a fresh electrode surface, whereby highly reproducible results can be obtained.

Even in the substantial absence of the retaining substance, a piston or the like is connected with the tube to produce a slight negative pressure in it, thereby holding the paste in the tube so that it can be used as a sensor.

In the electrode of the present invention, furthermore, as illustrated in Fig. 4, an end (base end) of the tube 11 is provided with a connecting port 15. A piston (syringe) 16 or a pump 17 is connected with the connecting port 15 via a tube (not shown) in order to feed a fresh carbon paste.

In the above-described construction, an enzyme or the like kneaded with carbon in a paste form using a solvent for preventing dispersion in the solution may be extruded to give a fresh carbon paste surface, thereby providing a carbon paste suspension type electrode or carbon paste disk electrode having a fresh electrode surface.

The carbon sensor electrode of the present invention with the above-mentioned construction has a structure in which a carbon paste containing thin carbon materials as an electric lead and preferably containing a reactant such as an enzyme is housed inside an insulating outer layer, and a tip portion degraded upon use is extruded and can be easily removed. Hence, the carbon sensor electrode can easily provide a fresh sensor electrode surface for a long term, thus ensuring highly practical use.

The present embodiment will be described in more detail below.

To graphite particles (mechanical pencil lead powder) was added liquid paraffin in an amount of 35% based on the amount of the graphite particles. The particles were thoroughly dispersed, and the mixture kneaded. Then, the resulting paste was poured into a syringe without a needle, and injected into an open-ended silicone tube with a diameter of 1.5 mm by means of the piston of the syringe.

An end of the tube with the paste fully introduced was cut off, and the surface of the electrode was flattened with a smooth sheet such as a waxed paper. Then, a dialysis membrane (Union Carbide) cut in a round shape with a slightly larger size than the electrode surface was placed on the tip of the tube, and fixed in place with an O-ring.

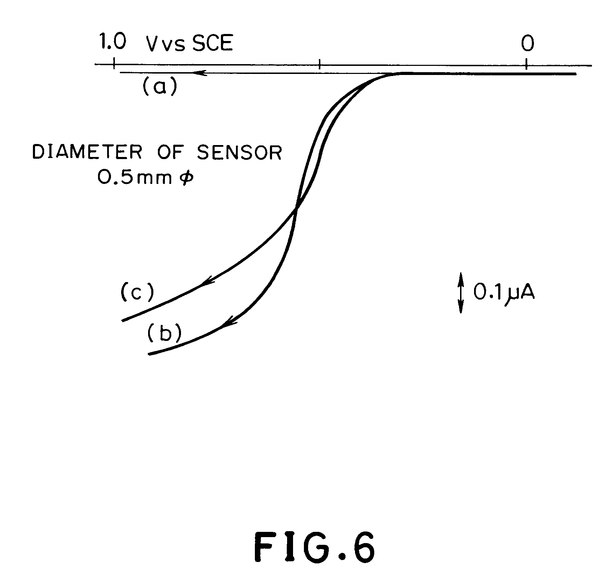

This electrode was used to detect uric acid in urine. Generally, the pH of human urine is about 6.5, and so a known amount of Ringer's solution adjusted to pH 6.5 containing a certain volume of human urine was used as an assay system. Lactam-type uric acid has three quinone groups (-C=0), one of which produces a rapid electrode reaction with the hydroxyl group -OH of the lactim form based on a redox reaction, thus showing an easily observable redox wave. Its redox potential is about 0.5 V vs. SCE at that pH, and reduction waves are at much more negative potentials. The results are given in Fig. 6. With the background Ringer's solution, no waves were observed as in (a) of the drawing. When 50 µg uric acid was contained in 1 ml of the solution, the curve (b) was obtained. The results (c) with uric acid in human urine were slightly less than in (b). The preparation of a calibration curve would make assay easy. When the electrode was dipped for more than about 5 minutes, the wave heights gradually decreased, probably because other ingredients in the urine may have been adsorbed to it. Since the adsorbate cannot be removed without potent oxidation, the paste electrode at the tip of the electrode once used should be cut off or extruded off, and next assay should be performed with a fresh electrode surface. Upon addition of much uric acid to human urine, sediments occur, hindering assay.

### Embodiment 4

In many natural enzyme reactions, hydrogen peroxide is generated after completion of the reaction. Thus, the progress of the enzyme reaction can be estimated by measuring the content of hydrogen peroxide in the reaction solution. In the present invention, the final objective is a sensor electrode of the present construction incorporating peroxidase contained in naturally occurring wasabi (a Japanese horseradish). Instead, the use of Iron-EDTA facilitates the detection of hydrogen peroxide, because of an electrochemical catalytic reaction as illustrated in Fig. 2.

Therefore, an imitation enzyme having Iron-EDTA saturated in an 80% aqueous solution of glycerin was kneaded with carbon, and the detection of hydrogen peroxide in a solution was attempted using this kneaded product. In detail, graphite particles (mechanical pencil lead powder) were thoroughly dispersed and kneaded in an 80% aqueous solution of glycerin (Wako Junyaku). Then, the resulting paste was poured into a syringe without a needle, and injected into an open-ended tube with a diameter of 0.5 mm by means of the piston of the syringe to prepare a carbon sensor electrode with a built-in imitation enzyme.

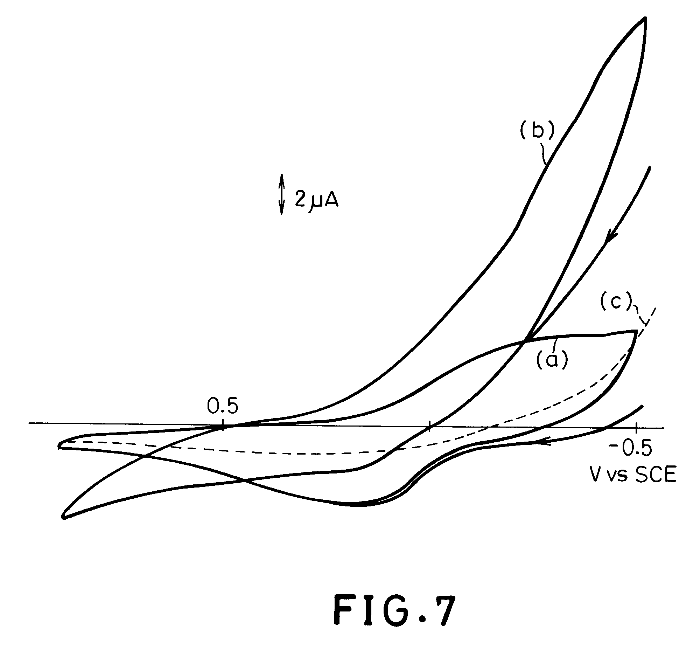

This electrode was used to detect 0.03% by volume of hydrogen peroxide present in acetate buffer solution (pH 5.5). The results are shown in Fig. 7. A curve (a) is a voltammogram of iron-EDTA determined by the Fe-EDTA-incorporated sensor electrode. A curve (b) is a voltammogram obtained in the solution containing 0.03% hydrogen peroxide with the use of this sensor electrode. One can clearly observe wave heights increasing because of hydrogen peroxide.

The detection of 0.03% by volume of hydrogen peroxide was attempted using the paste electrode without Fe-EDTA. The results are revealed as a curve (c).

As described above, the reactant-incorporated carbon sensor electrode of the present invention provides a disposable carbon sensor electrode suitable for in situ measurement of specific substances in many biological samples which has been infeasible for long with conventional sensors or sensor electrodes.

Specifically, the carbon sensor electrode using a carbon paste in accordance with the present invention serves as a practical sensor electrode which comprises a carbon paste incorporating a reactant such as an enzyme in an insulating tube. It has a structure capable of long-term use and can easily provide a fresh sensor electrode surface when its tip is either snapped or cut off, or extruded.

Moreover, the carbon sensor electrode using a composite carbon material in accordance with the present invention serves as a practical sensor electrode which comprises carbon fibers or the like incorporating a reactant such as an enzyme in an insulating tube. It has a structure capable of long-term use and can easily provide a fresh sensor electrode surface when its tip is either snapped or cut off.

The present invention has been described in detail with respect to preferred embodiments, and it will now be obvious that changes and modifications may be made without departing from the invention in its broader aspects, and it is our intention, therefore, in the appended claims to cover all such changes and modifications as fall within the true spirit of the invention.

The invention may be summarized as follows:
1. A carbon sensor electrode characterized by comprising:
   an insulating outer layer;
   a solution containing a reactant filled inside the insulating outer layer; and
   a carbon electrode inserted in the solution containing the reactant.
2. A carbon sensor electrode characterized in that the carbon electrode is a bundle of many carbon fibers put together.
3. A carbon sensor electrode characterized in that the carbon electrode is a bundle of many carbon rods put together.
4. A carbon sensor electrode characterized in that the insulating outer layer is an insulating tube.
5. A carbon sensor electrode characterized in that the insulating outer layer is composed of an insulating sheet.
6. A carbon sensor electrode of claims 1 to 5, characterized in that the reactant is an organic substance including an active enzyme, a nitrogen metabolite such as uric acid, an amino acid metabolite, a neurotransmitter such as dopamine, a vitamin, an organic acid such as acetic acid or citric acid, an amine, an alcohol, or a medicine such as an anesthetic; a complex ion; an inorganic acid such as hydrochloric acid or sulfuric acid; an alkali such as ammonia or hydroxide; a salt of the inorganic acid and alkali; a soluble gas such as oxygen, chlorine or nitrogen oxide; a mixture of any of these; or a substance containing any of them.
7. A carbon sensor electrode , characterized in that the solution containing the reactant is held in place by a carrier.
8. A carbon sensor electrode characterized in that the carrier is at least one of vaseline, paraffin, oil, glycerin, agar and gelatin.
9. A carbon sensor electrode characterized in that the tip opening of the insulating outer layer is sealed with an ion permeable membrane, an ion exchange membrane or a dialysis membrane.
10. A carbon sensor electrode characterized in that a reaction solution feeder for feeding a solution containing a reactant into the outer layer is connected with a base end portion of the outer layer.
11. A carbon sensor electrode characterized by comprising:
   an insulating outer layer; and
   a carbon paste filled inside the insulating outer layer;
   the carbon paste comprises at least a fine carbon material and a carrier.
12. A carbon sensor electrode characterized in that the fine carbon material is selected from activated carbon particles, graphite particles, amorphous carbon particles, short carbon fibers, and mixtures of these.
13. A carbon sensor electrode , characterized in that the carrier is at least one of vaseline, glycerin, oil, paraffin, agar and gelatin.
14. A carbon sensor electrode , characterized in that the carbon paste contains a reactant.
15. A carbon sensor electrode , characterized in that the reactant is an organic substance including an active enzyme, a nitrogen metabolite such as uric acid, an amino acid metabolite, a neurotransmitter such as dopamine, a vitamin, an organic acid such as acetic acid or citric acid, an amine, an alcohol, or a medicine such as an anesthetic; a complex ion; an inorganic acid such as hydrochloric acid or sulfuric acid; an alkali such as ammonia or hydroxide; a salt of the inorganic acid and alkali; a soluble gas such as oxygen, chlorine or nitrogen oxide; a mixture of any of these; or a substance containing any of them.
16. A carbon sensor electrode , characterized in that a dispersing medium is used to mix the reactant and the fine carbon material, and the dispersing medium is either an aqueous solution, an organic solvent, or a mixture of these.
17. A carbon sensor electrode , characterized in that the insulating outer layer is an insulating tube.
18. A carbon sensor electrode characterized in that the insulating outer layer is an insulating sheet.
19. A carbon sensor electrode characterized in that the tip opening of the insulating outer layer is sealed with an ion permeable membrane, an ion exchange membrane or a dialysis membrane.
20. A carbon sensor electrode , characterized in that a carbon paste feeder capable of continuously feeding the carbon paste into the outer layer is connected with a base end portion of the outer layer.
21. A process for producing a carbon sensor electrode characterized by comprising;
   dipping a carbon electrode in a solution containing a reactant; and
   covering the carbon electrode with an insulating outer layer with the solution being held in place to make the reactant coexistent in the periphery of the carbon electrode.
22. A process for producing a carbon sensor electrode , characterized in that the insulating outer layer is an insulating tube.
23. A process for producing a carbon sensor electrode , characterized in that the insulating outer layer is composed of an insulating sheet.
24. A process for producing a carbon sensor electrode characterized by comprising;
   pouring a solution containing a reactant into an insulating tube; and
   inserting a carbon electrode into the tube to impregnate the carbon electrode with the reactant, hold the reactant in place in the carbon electrode, and chemically modify the carbon electrode with the reactant, as well as to make the reactant coexistent in the periphery of the carbon electrode.
25. A process for producing a carbon sensor electrode characterized in that the carbon electrode is a bundle of many carbon fibers put together.
26. A process for producing a carbon sensor electrode characterized in that the carbon electrode is a bundle of many carbon rods put together.
27. A process for producing a carbon sensor electrode characterized in that the reactant is an organic substance including an active enzyme, a nitrogen metabolite such as uric acid, an amino acid metabolite, a neurotransmitter such as dopamine, a vitamin, an organic acid such as acetic acid or citric acid, an amine, an alcohol, or a medicine such as an anesthetic; a complex ion; an inorganic acid such as hydrochloric acid or sulfuric acid; an alkali such as ammonia or hydroxide; a salt of the inorganic acid and alkali; a soluble gas such as oxygen, chlorine or nitrogen oxide; a mixture of any of these; or a substance containing any of them.
28. A process for producing a carbon sensor electrode characterized in that a carrier is added to the solution containing the reactant to prevent the outflow of the reactant into the bulk solution.
29. A process for producing a carbon sensor electrode , characterized in that the carrier is at least one of vaseline, paraffin, oil, glycerin, agar and gelatin.
30. A process for producing a carbon sensor electrode characterized in that the carbon electrode is disposed in the insulating outer layer, and then the tip opening of the insulating outer layer is sealed with an ion permeable membrane.
31. A process for producing a carbon sensor electrode characterized in that a reaction solution feeder capable of continuously feeding a solution containing a reactant into the outer layer is connected with a base end portion of the outer layer.
32. A process for producing a carbon sensor electrode characterized by comprising:
   homogeneously mixing at least a fine carbon material and a carrier with a dispersing medium to prepare a carbon paste; and
   placing the carbon paste inside a tubular outer layer.
33. A process for producing a carbon sensor electrode , characterized in that the fine carbon material is selected from activated carbon particles, graphite particles, amorphous carbon particles, short carbon fibers, and mixtures of these.
34. A process for producing a carbon sensor electrode characterized in that the carrier is at least one of vaseline, glycerin, oil, paraffin, agar and gelatin.
35. A process for producing a carbon sensor electrode characterized in that the carbon paste contains a reactant.
36. A process for producing a carbon sensor electrode characterized in that the reactant is an organic substance including an active enzyme, a nitrogen metabolite such as uric acid, an amino acid metabolite, a neurotransmitter such as dopamine, a vitamin, an organic acid such as acetic acid or citric acid, an amine, an alcohol, or a medicine such as an anesthetic; a complex ion; an inorganic acid such as hydrochloric acid or sulfuric acid; an alkali such as ammonia or hydroxide; a salt of the inorganic acid and alkali; a soluble gas such as oxygen, chlorine or nitrogen oxide; a mixture of any of these; or a substance containing any of them.
37. A process for producing a carbon sensor electrode characterized in that either an aqueous solution, an organic solvent, or a mixture of these is used as a dispersing medium for mixing the reactant and the fine carbon material.
38. A process for producing a carbon sensor electrode characterized in that the insulating outer layer is an insulating tube.
39. A process for producing a carbon sensor electrode characterized in that the insulating outer layer is an insulating sheet.
40. A process for producing a carbon sensor electrode characterized in that the tip opening of the insulating outer layer is sealed with an ion permeable membrane or a dialysis membrane.
41. A process for producing a carbon sensor electrode characterized in that a carbon paste feeder capable of continuously feeding the carbon paste into the outer layer is connected with a base end portion of the outer layer.

## Claims

1. A carbon sensor electrode characterized by comprising:
an insulating outer layer;
a solution containing a reactant filled inside the insulating outer layer; and
a carbon electrode inserted in the solution containing the reactant.

2. A carbon sensor electrode as claimed in claim 1, characterized in that the carbon electrode is a bundle of many carbon fibers put together.

3. A carbon sensor electrode as claimed in claim 1, characterized in that the carbon electrode is a bundle of many carbon rods put together.

4. A carbon sensor electrode as claimed in any one of claims 1 to 3, characterized in that the insulating outer layer is an insulating tube.

5. A carbon sensor electrode as claimed in any one of claims 1 to 3, characterized in that the insulating outer layer is composed of an insulating sheet.

6. A carbon sensor electrode as claimed in any one of claims 1 to 5, characterized in that the reactant is an organic substance including an active enzyme, a nitrogen metabolite such as uric acid, an amino acid metabolite, a neurotransmitter such as dopamine, a vitamin, an organic acid such as acetic acid or citric acid, an amine, an alcohol, or a medicine such as an anesthetic; a complex ion; an inorganic acid such as hydrochloric acid or sulfuric acid; an alkali such as ammonia or hydroxide; a salt of the inorganic acid and alkali; a soluble gas such as oxygen, chlorine or nitrogen oxide; a mixture of any of these; or a substance containing any of them.

7. A carbon sensor electrode characterized by comprising:
an insulating outer layer; and
a carbon paste filled inside the insulating outer layer;
the carbon paste comprises at least a fine carbon material and a carrier.

8. A carbon sensor electrode as claimed in claim 7, characterized in that the fine carbon material is selected from activated carbon particles, graphite particles, amorphous carbon particles, short carbon fibers, and mixtures of these.

9. A process for producing a carbon sensor electrode characterized by comprising;
dipping a carbon electrode in a solution containing a reactant; and
covering the carbon electrode with an insulating outer layer with the solution being held in place to make the reactant coexistent in the periphery of the carbon electrode.

10. A process for producing a carbon sensor electrode as claimed in claim 9 , characterized in that the insulating outer layer is an insulating tube.

11. A process for producing a carbon sensor electrode characterized by comprising;
pouring a solution containing a reactant into an insulating tube; and
inserting a carbon electrode into the tube to impregnate the carbon electrode with the reactant, hold the reactant in place in the carbon electrode, and chemically modify the carbon electrode with the reactant, as well as to make the reactant coexistent in the periphery of the carbon electrode.

12. A process for producing a carbon sensor electrode characterized by comprising:
homogeneously mixing at least a fine carbon material and a carrier with a dispersing medium to prepare a carbon paste; and
placing the carbon paste inside a tubular outer layer.
